Europäisches Patentamt

European Patent Office

Office européen des brevets

(11) Publication number : **0 485 111 A2**

# EUROPEAN PATENT APPLICATION

(21) Application number : **91310059.0**

(22) Date of filing : **31.10.91**

(51) Int. Cl.⁵ : **A61K 31/47,** A61K 31/35,
// (A61K31/47, 31:405,
31:19), (A61K31/35, 31:405,
31:19)

(30) Priority : **06.11.90 EP 90403144**

(43) Date of publication of application :
**13.05.92 Bulletin 92/20**

(84) Designated Contracting States :
**AT BE CH DE DK ES FR GB GR IT LI LU NL SE**

(71) Applicant : **IMPERIAL CHEMICAL
INDUSTRIES PLC
Imperial Chemical House, Millbank
London SW1P 3JF (GB)**
(71) Applicant : **ICI PHARMA
Immeuble "Le Galien" B.P. 127 1 Rue des
Chauffours
F-95022 Cergy Cédex (FR)**

(72) Inventor : **Foster, Stephen John
Briar-Lea, Holmesville Avenue
Congleton, Cheshire, CW12 4HA (GB)**

(74) Representative : **Smith, Stephen Collyer et al
Imperial Chemical Industries PLC Legal
Department: Patents P.O. Box 6 Bessemer
Road
Welwyn Garden City Herts, AL7 1HD (GB)**

(54) **The use of lipoxygenase and cyclooxygenase inhibitors as synergistic agents.**

(57) The invention relates to synergistic agents for the treatment of inflammatory or arthritic conditions which comprise one of a selected group of inhibitors of the enzyme 5-lipoxygenase in conjunction or admixture with an inhibitor of the enzyme cyclooxygenase. Conveniently the 5-lipoxygenase inhibitor is, for example, 6-[(3-fluoro-5-[4-methoxy-3,4,5,6-tetrahydro-2H-pyran-4-yl]phenoxy)methyl]-1-methyl-2-quinolone or 4-[5-fluoro-3-(4-methyl-3-oxo-2,3-dihydro-4H-1,4-benzoxazin-7-ylthio)phenyl]-4-methoxytetrahydropyran and the cyclooxygenase inhibitor is selected from indomethacin, flurbiprofen and diclofenac. The invention also relates to a pharmaceutical composition containing said synergistic agent and to the use of said agent in the manufacture of a medicament for the synergistic treatment of inflammatory or arthritic disease.

EP 0 485 111 A2

The invention relates to a synergistic agent for the treatment of inflammatory or arthritic conditions. More particularly the invention relates to an agent comprising an inhibitor of the enzyme 5-lipoxygenase (hereinafter 5-LO) and an inhibitor of the enzyme cyclooxygenase (hereinafter CO) for the synergistic treatment of inflammatory or arthritic conditions. The invention also relates to a pharmaceutical composition containing said synergistic agent and to the use of said agent in the manufacture of a medicament for the synergistic treatment of inflammatory or arthritic disease.

The enzyme 5-LO is known to be involved in catalysing the oxidation of arachidonic acid to give rise via a cascade process to the physiologically active leukotrienes such as leukotriene $B_4$ ($LTB_4$) and the peptido-lipid leukotrienes such as leukotriene $C_4$ ($LTC_4$) and leukotriene $D_4$ ($LTD_4$) and various metabolites. Various inhibitors of 5-LO are known such as those disclosed in European Patent Applications Nos. 0375404 A2 and 0385662 A2.

The biosynthetic relationship and physiological properties of the leukotrienes are summarised by G.W. Taylor and S.R. Clarke in Trends in Pharmacological Sciences, 1986, 7, 100-1003. The leukotrienes and their metabolites have been implicated in the production and development of various inflammatory and allergic diseases such as arthritic diseases, asthma, allergic rhinitis, atopic dermatitis, psoriasis, cardiovascular and cerebrovascular disorders and inflammatory bowel disease. In addition the leukotrienes are mediators of inflammatory diseases by virtue of their ability to modulate lymphocyte and leukocyte function.

The enzyme CO in also known to be involved in catalysing the oxidation of arachidonic acid to give rise to the physiologically active prostaglandins and thromboxanes. Many inhibitors of CO are known such as the non-steroidal anti-inflammatory agents, for example, indomethacin, acetylsalicyclic acid, ibuprofen, sulindac, tolmetin or piroxicam.

It is disclosed in European Patent Applications Nos. 0375404 A2 and 0385662 A2 that compounds which are inhibitors of 5-LO have, by virtue of their effects on leukotriene production, certain cytoprotective effects, for example they are useful in reducing or suppressing certain of the adverse gastrointestinal effects of cyclooxygenase inhibitory non-steroidal anti-inflammatory agents. In addition it is disclosed that co-administration of a 5-LO inhibitor with a non-steroidal anti-inflammatory agent can result in a reduction of the quantity of the latter agent needed to produce a therapeutic effect, thereby reducing the likelihood of adverse side-effects. A pharmaceutical composition comprising a 5-LO inhibitor, a CO inhibitory non-steroidal anti-inflammatory agent and a diluent or carrier is also disclosed.

Thus there is a disclosure that a merely additive effect will be achieved when a 5-LO inhibitor and a CO-inhibitor are used together in the treatment of inflammatory or arthritic conditions. We have now surprisingly discovered that the combination of particular inhibitors of 5-LO and various cyclooxygenase inhibitory non-steroidal anti-inflammatory agents gives rise not merely to an additive effect but a synergistic effect. Thereby the likelihood of adverse side-effects is much reduced and the likelihood of effective treatment of the inflammatory or arthritic condition is increased.

According to the invention there is provided a synergistic agent for the treatment of inflammatory or arthritic conditions comprising a 5-lipoxygenase inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
in conjunction or admixture with a non-steroidal anti-inflammatory agent.

Conveniently the 5-lipoxygenase inhibitor is 6-[(3-fluoro-5-[4-methoxy-3,4,5,6-tetrahydro-2H-pyran-4-yl]phenoxy)methyl]-1-methyl-2-quinolone. Alternatively the 5-lipoxygenase inhibitor is conveniently 4-[5-fluoro-3-(4-methyl-3-oxo-2,3-dihydro-4H-1,4-benzoxazin-7-ylthio)phenyl]-4-methoxytetrahydropyran.

Suitable cyclooxygenase-inhibitory non-steroidal anti-inflammatory agents include, for example, arylacetic acid derivatives such as diclofenac, indomethacin, sulindac and tolmetin, arylpropionic acid derivatives such as fenoprofen, flurbiprofen, ibuprofen and ketoprofen, arylbutyric acid derivatives such as fenbufen, salicylic acid derivatives such as acetylsalicyclic acid, aminoarylcarboxylic acid derivatives such as flufenamic acid, and thiazinecarboxamides such as piroxicam. Preferably the non-steroidal anti-inflammatory agent is selected from indomethacin, flurbiprofen and diclofenac.

It will be appreciated that the 5-LO and CO inhibitors of the invention may be brought into admixture before dosing to the human or animal body or alternatively they may be used one in conjunction with the other i.e. not necessarily simultaneously but sequentially or separately. It will be appreciated that a synergistically-effective treatment may be attained even on the separate administration of the 5-LO or CO inhibitors as the synergistic effect will be dependent on the simultaneous presence of both agents at the inflammatory site. Factors such as the rate of absorption, metabolism and the rate of excretion of each agent will affect their presence at the inflammatory site. Such factors are routinely considered by, and are well within the ordinary skill of, the clinician when he contemplates the treatment of a medical condition which requires the conjoint administration of two agents in order to obtain a beneficial effect. Thus the synergistic agent of the invention is a product containing the 5-lipoxygenase inhibitor and a non-steroidal anti-inflammatory agent as a combined preparation for simultaneous, sequential or separate use in the synergistic treatment of inflammatory or arthritic conditions.

According to a particular aspect of the invention there is provided a synergistic agent for the treatment of inflammatory or arthritic conditions comprising the 5-lipoxygenase inhibitor 6-[(3-fluoro-5-[4-methoxy-3,4,5,6-tetrahydro-2H-pyran-4-yl]phenoxy)methyl]-1-methyl-2-quinolone in conjunction or admixture with a non-steroidal anti-inflammatory agent selected from indomethacin, flurbiprofen and diclofenac.

The 5-LO inhibitor 6-[(3-fluoro-5-[4-methoxy-3,4,5,6-tetrahydro-2H-pyran-4-yl]phenoxy)methyl]-1-methyl-2-quinolone is disclosed in European Patent Application No. 0385662 A2 (Example 6, Compound No 16 within Table II therein); and as the 8th of the 19 compounds listed on pages 15 and 16 therein, although the alternative chemical name of 4-[5-fluoro-3-(1,2-dihydro-1-methyl-2-oxoquinolin-6-ylmethoxy)phenyl]-4-methoxytetrahydropyran was used therein.

The amount of each active ingredient (that is the 5-LO inhibitor or a CO inhibitor) that is combined with the other active ingredient to produce the agent of the invention is such that a synergistic anti-inflammatory or anti-arthritic effect is obtained upon dosage of the agent. For example, a synergistic agent of the invention will generally contain, for example, from 0.5 mg to 2 g of each active ingredient. In particular the amount of the CO inhibitor ingredient of the synergistic agent of the invention will have as an upper limit the amount disclosed in the art for conventional monotherapy with such a compound.

In the synergistic agent of the invention the molar ratio of the two active ingredients will necessarily vary depending upon the CO-inhibitor selected and the intended application. The molar ratio will be selected such that a synergistic anti-inflammatory or anti-arthritic effect is obtained upon dosage of the agent. In particular the molar ratio may be in the range, for example, 100:1 to 1:100; more particularly in the range, for example, 10:1 to 1:10 ; and most particularly in the range 5:1 to 1:5.

It will be appreciated that the 5-LO and CO inhibitors of the invention may be brought into intimate admixture when a synergistic agent as defined hereinbefore is prepared. Alternatively the 5-LO and CO inhibitors of the invention may be dosed separately or sequentially. According to a further aspect of the invention there is provided a process for the preparation of a synergistic agent as defined hereinbefore characterised by bringing into conjunction or admixture a 5-LO inhibitor as defined hereinbefore and a non-steroidal anti-inflammatory agent as defined hereinbefore.

The synergistic effect obtainable by use of the agent of the present invention may be demonstrated using the standard in-vivo anti-inflammatory test set out below:-

The test is based upon the observation that single high doses of arachidonic acid when applied topically to an ear of a mouse induce the rapid onset of an inflammatory oedema which is believe to be associated with the infiltration of neutrophils (D. Aked et alia, Brit. J. Pharmacol, 1986, 89, 431). According to the test method, groups of 10 mice (25-30 g each) were dosed orally with vehicle alone (a mixture of 0.5% hydroxypropylmethylcellulose, 0.1% polysorbate 80 and water), with the 5-LO inhibitor alone, with a CO-inhibitor alone, or with a combination of 5-LO and CO inhibitors. After a period of 1 hour arachidonic acid (1 mg in 10 microlitres of acetone) was applied topically to one ear of each mouse. After a further period of 1 hour the mice were killed and a disc-shaped portion (6 mm in diameter) was punched from each ear. Comparison of the weights of the ear-discs between the arachidonic acid treated and untreated ears allows the effect of each treatment to be determined.

Details of the inhibition of the arachidonic acid induced inflammatory response are disclosed in the accompanying Examples 1 to 10. In general it is apparent therefrom that the anti-inflammatory effect of the 5-LO inhibitor, 6-[(3-fluoro-5-[4-methoxy-3,4,5,6-tetrahydro-2H-pyran-4-yl]phenoxy)methyl]-1-methyl-2-quinolone is variable (0 to 47% inhibition at 1-10 mg/kg orally), and that the anti-inflammatory effect of the CO inhibitors indomethacin, flurbiprofen or diclofenac is also variable (0 to 55% inhibition at 1-10 mg/kg orally); whereas there is a marked synergistic anti-inflammatory effect when the 5-LO inhibitor and a CO inhibitor are dosed in conjunction or admixture (19 to 88% inhibition at doses of 1-10 mg/kg orally). A similar effect is seen in Examples 11 to 15 relating to the use of other 5-lipoxygenase inhibitors of the invention.

No overt toxicity or other untoward effects are present when a synergistic agent as defined hereinbefore is administered at several multiples of the minimum inhibitory dose.

According to a convenient feature of the invention there is provided a synergistic agent as defined hereinbefore wherein the non-steroidal anti-inflammatory agent is indomethacin.

According to a further convenient feature of the invention there is provided a synergistic agent as defined hereinbefore wherein the non-steroidal anti-inflammatory agent is flurbiprofen.

According to a further convenient feature of the invention there is provided a synergistic agent as defined hereinbefore wherein the non-steroidal agent is diclofenac.

According to a futher feature of the present invention there is provided a pharmaceutical composition for the synergistic treatment of inflammatory or arthritic conditions which comprises a 5-lipoxygenase inhibitor selected from

6-[(3-fluoro-5-[4-methoxy-3,4,5,6-tetrahydro-2$\underline{H}$-pyran-4-yl]phenoxy)methyl]-1-methyl-2-quinolone,

4-[5-fluoro-3-(4-methyl-3-oxo-2,3-dihydro-4H-1,4-benzoxazin-7-ylthio)phenyl]-4-methoxytetrahydropyran,

4-methoxy-4-[5-(1-methyl-2-oxo-1,2,3,4-tetrahydroquinolin-6-ylthio)thien-2-yl]tetrahydropyran,

4-[5-fluoro-3-(1-methyl-2-oxo-1,2,3,4-tetrahydroquinolin-6-ylthio)phenyl]-4-methoxytetrahydropyran and

4-[5-fluoro-3-(4-(4-fluoro-alpha,alpha-difluorobenzyl)phenylthio)phenyl]-4-methoxytetrahydropyran,

in conjunction or admixture with a non-steroidal anti-inflammatory agent, and a pharmaceutically-acceptable diluent or carrier.

Conveniently the 5-lipoxygenase inhibitor is 6-[(3-fluoro-5-[4-methoxy-3,4,5,6-tetrahydro-2$\underline{H}$-pyran-4-yl]phenoxy)methyl]-1-methyl-2-quinolone. Alternatively the 5-lipoxygenase inhibitor is conveniently 4-[5-fluoro-3-(4-methyl-3-oxo-2,3-dihydro-4H-1,4-benzoxazin-7-ylthio)phenyl]-4-methoxytetrahydropyran.

Conveniently the non-steroidal anti-inflammatory agent is selected from indomethacin, flubiprofen and diclofenac.

According to a particular aspect of the present invention there is provided a pharmaceutical composition for the synergistic treatment of inflammatory or arthritic conditions which comprises the 5-lipoxygenase inhibitor 6-[(3-fluoro-5-[4-methoxy-3,4,5,6-tetrahydro-2$\underline{H}$-pyran-4-yl]phenoxy)methyl]-1-methyl-2-quinolone in conjunction or admixture with a non-steroidal anti-inflammatory agent selected from indomethacin, flubiprofen and diclofenac, and a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension, for nasal use, for example a snuff, nasal spray or nasal drops; for vaginal or rectal use, for example a suppository; for administration by inhalation, for example as a finely divided powder or a liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients.

It will be appreciated that the 5-LO and CO inhibitors of the invention may be brought into intimate admixture when a pharmaceutical composition is prepared in a conventional manner. Alternatively separate pharmaceutical compositions may be prepared, one containing the 5-LO inhibitor and the other containing a CO inhibitor. According to a further aspect of the invention there is provided a process for the preparation of a pharmaceutical composition for the synergistic treatment of inflammatory or arthritic conditions characterised by bringing into conjunction or admixture a 5-LO inhibitor as defined hereinbefore, a non-steroidal anti-inflammatory agent as defined hereinbefore and a pharmaceutically-acceptable diluent or carrier.

The amount of each active ingredient (that is the 5-LO inhibitor or a CO inhibitor) that is combined with the other active ingredient and one or more excipients to produce a single dosage form will necessarily vary depending upon the host treated and the particular route of administration. For example, a formulation intended for oral administration to humans will generally contain, for example, from 0.5 mg to 2 g of each active agent compounded with an appropriate and convenient amount of excipients which may vary from about 5 to about 98 percent by weight of the total composition. Dosage unit forms will generally contain about 1 mg to about 500 mg of each active ingredient. Alternatively each active ingredient may be combined separately with one or more excipients to produce a two-part dosage form. In the latter event the pharmaceutical composition of the invention comprises a kit comprising a first container with a suitable composition containing the 5-LO inhibitor and a second container with a suitable composition containing the CO-inhibitor. Such a kit may, for example, have the advantage that the physician wishing to obtain an anti-inflammatory or anti-arthritic effect can select the appropriate amounts of each active ingredient and the sequence and timing of the administration thereof. Those skilled in the art of treating warm-blooded animals can readily select the appropriate amounts of the active ingredients and the dosing schedule such that a synergistic effect is obtained. In particular the molar ratios set out hereinbefore and results set out hereinafter provide direction to the skilled person to this end.

According to a convenient feature of the invention there is provided a pharmaceutical composition as defined hereinbefore wherein the non-steroidal anti-inflammatory agent is indomethacin.

According to a further convenient feature of the invention there is provided a pharmaceutical composition as defined hereinbefore wherein the non-steroidal anti-inflammatory feature is flurbiprofen.

According to a further convenient aspect of the invention there is provided a pharmaceutical composition as defined hereinbefore wherein the non-steroidal anti-inflammatory agent is diclofenac.

As mentioned above a synergistic agent of the present invention is useful in the treatment of inflammatory or arthritic disease which is due alone or in part to the effects of metabolites of arachidonic acid arising by way of the linear (5-LO catalysed) and the cyclooxygenase pathways.

According to a further feature of the present invention there is provided the use of a 5-lipoxygenase inhibitor selected from

6-[(3-fluoro-5-[4-methoxy-3,4,5,6-tetrahydro-2H-pyran-4-yl]phenoxy)methyl]-1-methyl-2-quinolone, 4-[5-fluoro-3-(4-methyl-3-oxo-2,3-dihydro-4H-1,4-benzoxazin-7-ylthio)phenyl)-4-methoxytetrahydropyran, 4-methoxy-4-[5-(1-methyl-2-oxo-1,2,3,4-tetrahydroquinolin-6-ylthio)thien-2-yl]tetrahydropyran, 4-[5-fluoro-3-(1-methyl-2-oxo-1,2,3,4-tetrahydroquinolin-6-ylthio)phenyl]-4-methoxytetrahydropyran and 4-[5-fluoro-3-(4-(4-fluoro-alpha,alpha-difluorobenzyl)phenylthio)phenyl]-4-methoxytetrahydropyran, in conjunction or admixture with a non-steroidal anti-inflammatory agent in the manufacture of an agent for the synergistic treatment of inflammatory or arthritic disease.

The invention also concerns a method of treatment of inflammatory or arthritic disease which comprises administering to a warm-blooded animal requiring such treatment a synergistically-effective amount of a 5-lipoxygenase inhibitor selected from

6-[(3-fluoro-5-[4-methoxy-3,4,5,6-tetrahydro-2H-pyran-4-yl]phenoxy)methyl]-1-methyl-2-quinolone, 4-[5-fluoro-3-(4-methyl-3-oxo-2,3-dihydro-4H-1,4-benzoxazin-7-ylthio)phenyl)-4-methoxytetrahydropyran, 4-methoxy-4-[5-(1-methyl-2-oxo-1,2,3,4-tetrahydroquinolin-6-ylthio)thien-2-yl]tetrahydropyran, 4-[5-fluoro-3-(1-methyl-2-oxo-1,2,3,4-tetrahydroquinolin-6-ylthio)phenyl]-4-methoxytetrahydropyran and 4-[5-fluoro-3-(4-(4-fluoro-alpha,alpha-difluorobenzyl)phenylthio)phenyl]-4-methoxytetrahydropyran, and a non-steroidal anti-inflammatory agent.

Conveniently the 5-lipoxygenase inhibitor used in the manufacture of said agent or in said method is 6-[(3-fluoro-5-[4-methoxy-3,4,5,6-tetrahydro-2H-pyran-4-yl]phenoxy)methyl]-1-methyl-2-quinolone or 4-[5-fluoro-3-(4-methyl-3-oxo-2,3-dihydro-4H-1,4-benzoxazin-7-ylthio)phenyl]-4-methoxytetrahydropyran.

Conveniently the non-steroidal anti-inflammatory agent used in the manufacture of said agent or in said method of treatment is selected from indomethacin, flurbiprofen and diclofenac. Preferably indomethacin is used.

According to a particular aspect of the present invention there is provided a use or method as defined hereinbefore wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone and the non-steroidal anti-inflammatory agent is selected from indomethacin, flubiprofen and diclofenac.

The size of the dose for therapeutic or prophylactic purposes of an agent as defined above will naturally vary according to the nature and severity of the conditions, the age and sex of the animal or patient and the route of administration, according to well known principles of medicine.

In using a synergistic agent as defined above for therapeutic or prophylactic purposes it will generally be administered so that a daily dose in the range, for example, 0.5 mg to 75 mg per kg body weight of each active ingredient is received, given if required in divided doses. In general lower doses will be administered when a parenteral route is employed. Thus, for example, for intravenous administration, a dose of each active ingredient in the range, for example, 0.5 mg to 30 mg per kg body weight will generally be used. Similarly, for administration by inhalation, a dose of each active ingredient in the range, for example, 0.5 mg to 25 mg per kg body weight will be used. Preferably the oral route will be used so that a daily dose in the range, for example, 0.5 mg to 30 mg per kg body weight of each active ingredient is received.

The invention will now be illustrated in the following non-limiting Examples 1 to 10 which relate to the testing of synergistic agents of the invention in the mouse ear inflammatory model described above.

## Abbreviations:-

Within Examples 1 to 10 the 5-LO inhibitor 6-[(3-fluoro-5-[4-methoxy-3,4,5,6-tetrahydro-2H-pyran-4-yl]phenoxy)methyl]-1-methyl-2-quinolone is identified by the number [1]; indomethacin is identified by the letter I; flurbiprofen is identified by the letter F; and diclofenac is identified by the letter D.

The effect of each test compound or combination of compounds is calculated as follows ('ear weight diff.' means the mean difference in the weights of the disc-shaped portions of ear punched from the arachidonic acid-treated ear compared to the untreated ear for each mouse):-

$$\text{\% Inhibition} = \frac{(\text{Control Ear Weight Diff. - Test Ear Weight Diff.})}{\text{Control Ear Weight Diff.}} \times 100$$

| Ex | Agent (mg/kg) | % Inhib. | Comb. | % Inhib. | Synergistic Effect (%) |
|----|----|----|----|----|----|
| 1 | [1] | | [1] | | |
| | | | + I (10 mg/kg) | | |
| | 0 | 0 | 0 | 16.9 | |
| | 1 | 14.6 | 1 | 29.2 | − 2.3 |
| | 3 | 19.1 | 3 | 67.4 | + 31.4 |
| | 10 | 47.2 | 10 | 87.6 | + 23.5 |
| 2 | [1] | | [1] | | |
| | | | + I (10 mg/kg) | | |
| | 0 | 0 | 0 | 27.4 | |
| | 1 | − 5.3 | 1 | 38.9 | + 16.8 |
| | 3 | 9.5 | 3 | 44.3 | + 7.4 |
| | 10 | 0.5 | 10 | 70.5 | + 12.6 |
| 3 | [1] | | [1] | | |
| | | | + I (3 mg/kg) | | |
| | 0 | 0 | 0 | 12.8 | |
| | 1 | − 9.2 | 1 | 25.7 | + 22.1 |
| | 3 | 0 | 3 | 33.0 | + 20.2 |
| | 10 | 15.6 | 10 | 53.2 | + 24.8 |
| 4 | I | | I | | |
| | | | + [1] (10 mg/kg) | | |
| | 0 | 0 | 0 | 44.4 | |
| | 1 | − 8.3 | 1 | 20.8 | − 15.3 |
| | 3 | 15.3 | 3 | 77.8 | + 18.1 |
| | 10 | 55.3 | 10 | 79.2 | − 20.7 |

| Ex | Agent (mg/kg) | % Inhib. | Comb. | % Inhib. | Synergistic Effect (%) |
|---|---|---|---|---|---|
| 5 | I | | I + [1] (10 mg/kg) | | |
| | 0 | 0 | 0 | 15.2 | |
| | 1 | − 5.1 | 1 | 52.5 | + 41.4 |
| | 3 | 18.2 | 3 | 75.8 | + 42.4 |
| | 10 | 55.6 | 10 | 83.8 | + 13.0 |
| 6 | [1] | | [1] + F (3 mg/kg) | | |
| | 0 | 0 | 0 | 14.6 | |
| | 1 | 16.7 | 1 | 35.4 | + 4.1 |
| | 3 | 20.8 | 3 | 50.0 | + 14.6 |
| | 10 | 15.6 | 10 | 55.2 | + 20.0 |
| 7 | [1] | | [1] + F (3 mg/kg) | | |
| | 0 | 0 | 0 | 7.6 | |
| | 1 | 16.2 | 1 | 19.0 | − 4.8 |
| | 3 | 13.3 | 3 | 25.7 | + 4.8 |
| | 10 | 11.4 | 10 | 44.8 | + 25.8 |
| 8 | D | | D + [1] (10 mg/kg) | | |
| | 0 | 0 | 0 | 12.5 | |
| | 3 | 11.1 | 3 | 51.1 | + 27.5 |
| | 10 | − 3.4 | 10 | 36.4 | + 27.3 |
| | 30 | 11.4 | 30 | 64.8 | + 40.9 |

| Ex | Agent (mg/kg) | % Inhib. | Comb. | % Inhib. | Synergistic Effect (%) |
|----|------|------|------|------|------|
| 9 | D | | D | | |
| | | | + [1] (10 mg/kg) | | |
| | 0 | 0 | 0 | 25.7 | |
| | 3 | 5.9 | 3 | 57.4 | + 25.8 |
| | 10 | 4.9 | 10 | 63.4 | + 32.8 |
| | 30 | 17.8 | 30 | 71.3 | + 27.8 |
| 10 | D | | D | | |
| | | | + [1] (10 mg/kg) | | |
| | 0 | 0 | 0 | 3.2 | |
| | 3 | − 2.1 | 3 | 48.4 | + 49.5 |
| | 10 | 7.4 | 10 | 50.5 | + 39.9 |
| | 30 | 10.5 | 30 | 68.4 | + 44.7 |

The invention will now be illustrated further in the following non-limiting Examples 11 to 15 which relate to the testing of synergistic agents of the invention in the mouse ear inflammatory model.

## Abbreviations:-

Within Examples 11 to 15 indomethacin is identified by the letter I; the 5-LO inhibitor 4-[5-fluoro-3-(4-methyl-3-oxo-2,3-dihydro-4H-1,4-benzoxazin-7-ylthio)phenyl]-4-methoxytetrahydropyran is identified by the number [2]; the 5-LO inhibitor 4-methoxy-4-[5-(1-methyl-2-oxo-1,2,3,4-tetrahydroquinolin-6-ylthio)thien-2-yl]tetrahydropyran is identified by the number [3]; the 5-LO inhibitor 4-[5-fluoro-3-(1-methyl-2-oxo-1,2,3,4-tetrahydroquinolin-6-ylthio)phenyl]-4-methoxytetrahydropyran is identified by the number [4]; and the 5-LO inhibitor 4-{5-fluoro-3-(4-(4-fluoro-alpha,alpha-difluorobenzyl)phenylthio)phenyl]-4-methoxytetrahydropyran is identified by the number [5].

| Ex | Agent (mg/kg) | % Inhib. | Comb. | % Inhib. | Synergistic Effect (%) |
|----|------|------|------|------|------|
| 11 | [2] | | [2] | | |
| | | | + I (10 mg/kg) | | |
| | 0 | 0 | 0 | 30 | |
| | 3 | 15 | 3 | 50 | + 5 |
| | 10 | 22 | 10 | 17* | - 35 |
| | 30 | 34 | 30 | 84 | + 20 |
| 12 | I | | I | | |
| | | | + [2] (30 mg/kg) | | |
| | 0 | 0 | 0 | 27 | |
| | 1 | 13 | 1 | 57 | + 17 |
| | 3 | 27 | 3 | 74 | + 20 |
| | 10 | 33 | 10 | 82 | + 22 |
| 13 | [3] | | [3] | | |
| | | | + I (10 mg/kg) | | |
| | 0 | 0 | 0 | 10* | |
| | 3 | 17* | 3 | 45 | + 18 |
| | 10 | 15 | 10 | 75 | + 50 |
| | 30 | 20* | 30 | 83 | + 53 |
| 14 | [4] | | [4] | | |
| | | | + I (10 mg/kg) | | |
| | 0 | 0 | 0 | -2.5* | |
| | 3 | 5* | 3 | 45 | + 43 |
| | 10 | 21 | 10 | 76 | + 58 |
| | 30 | 22 | 30 | 71 | + 52 |

| Ex | Agent (mg/kg) | % Inhib. | Comb. | % Inhib. | Synergistic Effect (%) |
|---|---|---|---|---|---|
| 15 | [5] | | [5] + I (10 mg/kg) | | |
| | 0 | 0 | 0 | 27 | |
| | 3 | 11* | 3 | 30 | – 8 |
| | 10 | 16* | 10 | 57 | + 14 |
| | 30 | 17* | 30 | 57 | + 13 |

Notes

All of the % Inhibition figures are statistically significant except those indicated with an asterisk (*).

The 5-LO inhibitor 4-[5-fluoro-3-(4-methyl-3-oxo-2,3-dihydro-4H-1,4-benzoxazin-7-ylthio)phenyl]-4-methoxytetrahydropyran is disclosed in copending European Patent Application No. 91305532.3 (Example 5 thereof) by way of the following method:-

Butyl-lithium (1.6M in hexane, 1 ml) was added dropwise to a mixture of 7-mercapto-4-methyl-3-oxo-2,3-dihydro-4H-1,4-benzoxazine (0.292 g), 4-(3,5-difluorophenyl)-4-methoxytetrahydropyran (0.342 g) and N-methylpyrrolidin-2-one (3.75 ml) which had been cooled in an ice-bath. The mixture was stirred and allowed to warm to ambient temperature. The mixture was heated to 145°C for 90 minutes, the hexane being distilled out of the reaction mixture. The mixture was cooled to ambient temperature and partitioned between ethyl acetate and water. The organic phase was washed with water and with N aqueous sodium hydroxide solution, dried ($MgSO_4$) and evaporated. The residue was purified by column chromatography using a 15:1 v/v mixture of methylene chloride and diethyl ether as eluent. There was thus obtained 4-[5-fluoro-3-(4-methyl-3-oxo-2,3-dihydro-4H-1,4-benzoxazin-7-ylthio)phenyl]-4-methoxytetrahydropyran (0.202 g, 33%), m.p. 131-133°C.
NMR Spectrum ($CDCl_3$, δ values) 1.8-2.1 (m, 4H), 2.98 (s, 3H), 3.37 (s, 3H), 3.75-3.95 (m, 4H), 4.63 (s, 2H), 6.76-7.25 (m, 6H).

The 7-mercapto-4-methyl-3-oxo-2,3-dihydro-4H-1,4-benzoxazine used as a starting material was obtained as follows:-

A mixture of 5-fluoro-2-nitrophenol (10.05 g), potassium carbonate (10.6 g) and acetone (125 ml) was heated to reflux for 10 minutes. The mixture was cooled to ambient temperature and a solution of ethyl bromoacetate (7.8 ml) in acetone (10 ml) was added dropwise. The mixture was heated to reflux for 2.5 hours. The mixture was evaporated and the residue was partitioned between diethyl ether and water. The organic phase was washed with water, dried ($MgSO_4$) and evaporated. There was thus obtained ethyl 2-(5-fluoro-2-nitrophenoxy)acetate (14.28 g, 92%), m.p 44-46°C.

A mixture of ethyl 2-(5-fluoro-2-nitrophenoxy)acetate (11 g), benzylmercaptan (5.2 g), triethylamine (5.08 g) and DMF (50 ml) was stirred and heated to 80°C for 7 hours. The mixture was cooled, poured into water and acidified by the addition of dilute aqueous hydrochloric acid. The mixture was extracted with diethyl ether. The organic phase was washed with water and with brine, dried ($MgSO_4$) and evaporated to give ethyl 2-(5-benzylthio-2-nitrophenoxy)acetate (10.6 g, 68%) as a solid.

A mixture of a portion (8.68 g) of the product so obtained, stannous chloride dihydrate (Tet.Let., 1984, 839; 28.1 g), ethyl acetate (5 ml) and ethanol (50 ml) was heated to reflux for 30 minutes. The mixture was poured onto ice and a saturated aqueous sodium bicarbonate solution was added. The resultant precipitate was removed by filtration and the filtrate was extracted with ethyl acetate. The organic phase was washed with water and with brine, dried ($MgSO_4$) and evaporated to give 7-benzylthio-3-oxo-2,3-dihydro-4H-1,4-benzoxazine (3.32 g, 49%), m.p. 153-154°C.

A portion of (2.7 g) of the product so obtained was added to a stirred suspension of sodium hydride (60% w/w dispersion in mineral oil, 0.52 g; the oil was removed by washing the solid dispersion with petroleum ether) in DMF (10 ml) and the mixture was stirred at ambient temperature for 30 minutes. Methyl iodide (2.13 g) was added and the mixture was stirred at ambient temperature for 30 minutes. The mixture was partitioned between diethyl ether and water. The organic phase was washed with water and with brine, dried (MgSO₄) and evaporated to give 7-benzylthio-4-methyl-3-oxo-2,3-dihydro-4H-1,4-benzoxazine (2.6 g, 91%) as a solid.

A solution of 3-chloroperbenzoic acid (1.72 g) in chloroform (10 ml) was added dropwise to a solution of a portion (2 g) of the benzoxazine so obtained in chloroform (15 ml) which had been cooled to 0°C and the mixture was stirred at 0°C for 4 hours. Calcium hydroxide (0.74 g) was added and the mixture was stirred at ambient temperature for 15 minutes. The mixture was filtered and the filtrate was evaporated to give 7-benzylsulphinyl-4-methyl-3-oxo-2,3-dihydro-4H-1,4-benzoxazine (2.1 g) as a solid which was used without further purification.

Trifluoroacetic acid (4.2 g) was added dropwise to a stirred suspension of a portion (1.5 g) of the benzoxazine so obtained in methylene chloride (45 ml) and the solution so obtained was stirred at ambient temperature for 30 minutes and then heated to reflux for 30 minutes. The mixture was evaporated and the residue was partitioned between ethyl acetate and water. The organic phase was washed with water and with a saturated aqueous sodium bicarbonate solution, dried (MgSO₄) and evaporated. The residue was purified by column chromatography using increasingly polar mixtures of methylene chloride and diethyl ether as eluent. There was thus obtained di-(4-methyl-3-oxo-2,3-dihydro-4H-1,4-benzoxazin-7-yl) disulphide (0.68 g, 60%), m.p. 133-135°C.

After repetition of the preceding step triphenylphosphine (0.576 g) was added to a suspension of the disulphide (0.776 g) in 1,4-dioxane (9 ml). Water (2.5 ml) and concentrated hydrochloric acid (1 drop) were added in turn and the mixture was heated to 50°C for 1 hour. The mixture was cooled to ambient temperature and partitioned between ethyl acetate and 0.5 N aqueous sodium hydroxide solution. The organic phase was washed with water and with brine, dried (MgSO₄) and evaporated. There was thus obtained the required starting material (0.425 g, 55%) m.p. 95-96°C.

The 4-(3,5-difluorophenyl)-4-methoxytetrahydropyran used as a starting material was obtained as follows:-

A Grignard reagent was prepared from 3,5-difluorobromobenzene (38.6 g) and magnesium (4.88 g) in a mixture of toluene (100 ml) and THF (50 ml) using the following method. The 3,5-difluorobromobenzene was dissolved in toluene (50 ml) and a portion (aprox. 5%) of the solution was added to a stirred suspension of the magnesium in a mixture of toluene (50 ml) and THF (50 ml). The mixture was stirred at ambient temperature for approximately 40 minutes until the initiation of the exothermic formation of the Grignard reagent was observed. The mixture was cooled in an ice-bath to a temperature in the range 15 to 20°C while the remainder of the solution of 3,5- difluorobromobenzene was added. The mixture was stirred at ambient temperature for 2 hours.

Tetrahydropyran-4-one (10.69 g) was added over 1 hour to a portion (100 ml) of the Grignard reagent so obtained which was cooled to a temperature in the range 15 to 20°C. The mixture was stirred at ambient temperature for 2 hours. The mixture was cooled in an ice-bath and aqueous hydrochloric acid solution (50% w/v, 25 ml) and brine (30% w/v, 52 ml) were added in turn. The toluene layer was separated and the aqueous layer was extracted with toluene (32 ml). The organic solutions were combined and washed with water (4 x 32 ml). The solution was evaporated under reduced pressure to a volume of 16.3 ml. There was thus obtained a concentrated (90% w/v) solution of 4-(3,5-difluorophenyl)-4-hydroxytetrahydropyran in toluene. The concentrate was warmed to 60°C and chlorobenzene (22.25 ml) was added, the temperature being maintained at 60°C. The mixture was allowed to cool to ambient temperature and then cooled in an ice-bath to a temperature in the range 0 to 5°C. The precipitate was isolated and washed with hexane (2 x 10 ml). There was thus obtained 4-(3,5-difluorophenyl)-4-hydroxytetrahydropyran (12.2 g).

A portion (7.15 g) of the material so obtained was dissolved in N-methylpyrrolidin-2-one (25 ml) and added to a slurry of sodium hydride (60% w/w dispersion in mineral oil; 3.34 g) in N-methylpyrrolidin-2-one (32 ml) which was cooled in an ice-bath to approximately 20°C. The mixture was stirred at this temperature for 30 minutes. Methyl idodide (5.22 g) was dissolved in N-methylpyrrolidin-2-one (2 ml) and added to the mixture. The resultant mixture was warmed to 30°C and stirred for 2 hours. The mixture was evaporated. There was thus obtained 4-(3,5-difluorophenyl)-4-methoxytetrahydropyran which was used without further purification.

The 5-LO inhibitor 4-methoxy-4-[5-(1-methyl-2-oxo-1,2,3,4-tetrahydroquinolin-6-ylthio)thien-2-yl]tetrahydropyran is disclosed in copending European Patent Application No. 91305531.5 (Example 7 thereof) by way of the following method:-

A solution of 4-(5-bromothien-2-yl)-4-methoxytetrahydropyran (0.415 g) in THF (8 ml) was added dropwise to n-butyl-lithium (1.5M in hexane, 1 ml) which had been cooled to -78°C and the mixture was stirred at -78°C for 2 hours. A solution di-(1-methyl-2-oxo-1,2,3,4-tetrahydroquinolin-6-yl) disulphide (0.576 g) in THF (1.5 ml) was added and the mixture was stirred at -78°C for 3 hours. The mixture was allowed to warm to ambient temperature and was stirred for 16 hours. The mixture was poured into a cold aqueous ammonium chloride solution (15% w/v) and extracted with ethyl acetate. The organic phase was washed with water, dried (MgSO4) and evaporated. The residue was purified by column chromatography using initially a 6:4 v/v mixture of petroleum ether (b.p. 40-60°C) and ethyl acetate as eluent. There was thus obtained 4-methoxy-4-[5-(1-methyl-2-oxo-1,2,3,4-tetrahydroquinolin-6-ylthio)thien-2-yl]tetrahydropyran in 55% yield, m.p. 84-86°C.

NMR Spectrum (CD$_3$COCD$_3$, δ values) 1.95-2.20 (m, 4H), 2.4-3.0 (m, 4H), 3.06 (s, 3H), 3.27 (s, 3H), 3.65-3.80 (m, 4H), 7.0-7.3 (m, 5H).

The 4-(5-bromothien-2-yl)-4-methoxytetrahydropyran used as a starting material was obtained as follows:-

n-Butyl-lithium (1.6M in hexane, 31 ml) was added dropwise to a stirred solution of 2,5-bromothiophene (12.1 g) in THF (40 ml) which had been cooled to -40°C. The mixture was allowed to warm to -20°C and was stirred at this temperature for 1 hour. The mixture was cooled to -78°C and tetrahydropyran-4-one (5 g) was added dropwise. The mixture was allowed to warm to ambient temperature and was stirred for 12 hours. The mixture was poured into a cold aqueous ammonium chloride solution (15% w/v) and extracted with ethyl acetate. The organic phase was washed with water, dried (MgSO$_4$) and evaporated. The residue was purified by column chromatography using a 17:3 v/v mixture of methylene chloride and diethyl ether as eluent. There was thus obtained 4-(5-bromothien-2-yl)-4-hydroxytetrahydropyran (4.16 g, 32%), m.p. 100-102°C.

Sodium hydride (60% w/v dispersion in mineral oil, 0.72 g) was added portionwise to a solution of a portion (2.48 g) of the product so obtained in THF (30 ml) and the mixture was stirred at ambient temperature for 30 minutes. Methyl iodide (2.4 ml) was added and the mixture was stirred at ambient temperature for 18 hours. The mixture was poured into a cold saturated aqueous ammonium chloride solution and extracted with ethyl acetate. The organic phase was dried (MgSO$_4$) and evaporated. The residue was purified by column chromatography using a 9:1 v/v mixture of petroleum ether (b.p. 60-80°C) and ethyl acetate as eluent. There was thus obtained the required starting material (2.1 g, 80%), m.p. 57-59°C.

NMR Spectrum (CDCl$_3$, δ values) 1.9-2.2 (m, 4H), 3.06 (s, 3H), 3.6-3.85 (m, 4H), 6.87 (d, 1H), 7.03 (d, 1H).

The di-(1-methyl-2-oxo-1,2,3,4-tetrahydroquinolin-6-yl) disulphide used as a starting material was obtained as follows:-

A mixture of 1-methyl-1,2-dihydroquinolin-2-one (8 g), 10% palladium-on-charcoal catalyst (2 g) and ethanol (60 ml) was stirred under a pressure of 3.5 atmospheres of hydrogen for 24 hours. The mixture was filtered and evaporated. The residue was purified by column chromatography using a 9:1 v/v mixture of methylene chloride and diethyl ether as eluent. There was thus obtained 1-methyl-1,2,3,4-tetrahydroquinolin-2-one (7.88 g, 98%) as an oil.

A portion (1.6 g) of the product so obtained was added dropwise to chlorosulphonic acid (8 ml) and the mixture was heated to 80°C for 2.5 hours. The mixture was cooled to ambient temperature, poured onto a mixture of ice and water and extracted with ethyl acetate. The organic phase was washed with water, dried (MgSO$_4$) and evaporated to give 1-methyl-2-oxo-1,2,3,4-tetrahydroquinolin-6-ylsulphonyl chloride (1.97 g, 76%), m.p. 137-139°C.

NMR Spectrum (CDCl$_3$, δ values) 2.7-3.25 (m, 4H), 3.41 (s, 3H), 7.13 (d, 1H), 7.80-8.10 (m, 2H).

Trimethylsilyl iodide (5 g) was added dropwise to a solution of a portion (1.2 g) of the product so obtained in methylene chloride (40 ml) and the mixture was stirred at ambient temperature for 16 hours. The mixture was washed with a saturated aqueous sodium bicarbonate solution and with a saturated aqueous sodium sulphite solution, dried (MgSO$_4$) and evaporated. The residue was purified by column chromatography using a 9:1 v/v mixture of methylene chloride and diethyl ether as eluent. There was thus obtained the required disulphide (0.418 g, 43%), m.p. 130°C.

NMR Spectrum (CD$_3$COCD$_3$) 2.40-3.0 (m, 4H), 3.26 (s, 3H), 6.84 (d, 1H), 7.15-7.40 (m, 2H).

The 5-LO inhibitor 4-[5-fluoro-3-(1-methyl-2-oxo-1,2,3,4-tetrahydroquinolin-6-ylthio)phenyl]-4-methoxytetrahydropyran is obtained as follows:-

n-Butyl-lithium (1.4M in hexane, 1.43 ml) was added dropwise to a stirred solution of 6-mercapto-1-methyl-1,2,3,4-tetrahydroquinolin-2-one (0.386 g) and 4-(3,5-difluorophenyl)-4-methoxytetrahydropyran (0.547 g) in 1-methylpyrrolidin-2-one (6 ml) which had been cooled to 0°C. The mixture was heated to 145°C for 2 hours, the hexane being allowed to distil out from the reaction mixture. The mixture was cooled to ambient temperature and partitioned between ethyl acetate and water. The organic phase was washed with water, with 0.5N aqueous sodium hydroxide solution, with water and with brine, dried (MgSO$_4$) and evaporated. The residue was purified by column chromatography using increasingly polar mixtures of methylene chloride and diethyl ether as eluent. There was thus obtained 4-[5-fluoro-3-(1-methyl-2-oxo-1,2,3,4-tetrahydroquinolin-6-ylthio)phenyl]-4-methoxytetrahydropyran (0.404 g, 50%), m.p. 124-125°C.

The 6-mercapto-1-methyl-1,2,3,4-tetrahydroquinolin-2-one used as a starting material was obtained as follows:-

Water (1 ml) and concentrated hydrochloric acid (1 drop) were added in turn to a stirred suspension of di-(1-methyl-2-oxo-1,2,3,4-tetrahydroquinolin-6-yl) disulphide (0.576 g), triphenylphosphine (0.432 g) and 1,4-dioxane (5 ml) and the mixture was stirred at ambient temperature for 1 hour. The mixture was partitioned between ethyl acetate and 0.5N aqueous sodium hydroxide solution. The aqueous layer was washed with diethyl ether, acidified by the addition of dilute aqueous hydrochloric acid and extracted with ethyl acetate. The organic layer was washed with brine, dried (MgSO₄) and evaporated. The resultant oil crystallised on trituration under a mixture of petroleum ether (b.p. 60-80°C) and diethyl ether. There was thus obtained the required starting material (0.465 g, 80%), m.p. 84°C.

The 5-LO inhibitor 4-[5-fluoro-3-(4-(4-fluoro-alpha,alpha-difluorobenzyl)phenylthio)phenyl]-4-methoxytetrahydropyran is obtained as follows:-

A mixture of difluoro-4-fluoro-4'-iododiphenylmethane (8 g), 4-(5-fluoro-3-mercaptophenyl)-4-methoxytetrahydropyran (6.2 g), cuprous chloride (0.34 g), potassium carbonate (6.4 g) and DMF (22 ml) was heated to 120°C for 60 minutes. The mixture was cooled to ambient temperature and partitioned between water and diethyl ether. The organic phase was washed with water and with brine, dried (MgSO₄) and evaporated. The residue was purified by medium pressure liquid chromatography using increasingly polar mixtures of hexane and ethyl acetate as eluent. There was thus obtained 4-[5-fluoro-3-(4-(4-fluoro-alpha,alpha-difluorobenzyl)phenylthio)phenyl]-4-methoxytetrahydropyran (9 g, 85%), m.p. 54-57°C (recrystallised from a mixture of hexane and diethyl ether).

The difluoro-4-fluoro-4'-iododiphenylmethane used as a starting material was obtained as follows:-

A solution of 1,4-diiodobenzene (12 g) in THF (120 ml) was cooled to -80°C under an atmosphere of argon and n-butyl lithium (1.6M in hexane, 23 ml) was added dropwise. The mixture was stirred at -80°C for 30 minutes and then 4-fluorobenzaldehyde (4.6 g) was added dropwise. The mixture was stirred at -80°C for 30 minutes. Brine (100 ml) was added and the mixture was allowed to warm to ambient temperature. The mixture was extracted with diethyl ether. The organic phase was dried (MgSO₄) and evaporated to give 4-fluoro-4'-iododiphenylmethanol as an oil (13.4 g) which was used without further purification.

Pyridinium chlorochromate (6 g) was added to a mixture of 4-fluoro-4'-iododiphenylmethanol (6.7 g), silica (10 g) and methylene chloride (60 ml) and the mixture was stirred at ambient temperature for 60 minutes. Diethyl ether (20 ml) was added and the mixture was filtered. The filtrate was evaporated and the residue was recrystallised from a mixture of hexane and ethyl acetate. There was thus obtained 4-fluoro-4'-iodobenzophenone (3.9 g).

NMR Spectrum 7.1-7.3(m, 2H), 7.5(m, 2H), 7.7-7.9(m, 4H).

Ethane-1,2-dithiol (1.14 g) and boron trifluoride diacetate (1.14 g) were added in turn to a solution of 4-fluoro-4'-iodobenzophenone (2 g) in methylene chloride (5 ml) and the mixture was stirred at ambient temperature for 1 hour. Hexane (20 ml) and diethyl ether (5 ml) were added and the solution was extracted with 1N sodium hydroxide solution (3 x 20 ml). The organic phase was washed with water, dried (MgSO₄) and evaporated. The residue was purified by medium pressure liquid chromatography using increasingly polar mixtures of hexane and ethyl acetate as eluent. There was thus obtained 2-(4-fluorophenyl)-2-(4-iodophenyl)-1,3-dithiolane as an oil (2.3 g).

NMR Spectrum 3.45(s, 4H), 7.0(m, 2H), 7.4(d, 2H), 7.5-7.7(m, 4H).

Hydrogen fluoride-pyridine complex (1 ml) and a portion (0.8 g) of the dithiolane so obtained were added in turn to a solution of 1,3-dibromo-4,4-dimethyl-2,5-dioxoimidazolidine (0.58 g) in methylene chloride (5 ml) which had been cooled to -80°C. The mixture was stirred at -80°C for 20 minutes. Hexane (30 ml) was added and the mixture was allowed to warm to ambient temperature. The mixture was filtered through a column of alumina using increasingly polar mixtures of hexane and diethyl ether as eluent. The product so obtained was purified by medium pressure liquid chromatography on silica using increasingly polar mixture of hexane and ethyl acetate as eluent. There was thus obtained the required starting material as an oil (0.25 g).

NMR Spectrum 7.0-7.3(m, 4H), 7.45(m, 2H), 7.8(m, 2H).

The 4-(5-fluoro-3-mercaptophenyl)-4-methoxytetrahydropyran used as a starting material is described in European Patent Application No. 0420511 (Example 4 thereof).


## Example 16

The following illustrate representative pharmaceutical dosage forms containing either one of the two active ingredients of the synergistic agent of the invention [hereinafter compound X is used to identify one of the 5-LO inhibitors [1] to [5] or one of the CO inhibitors I, F or D (using the abbreviations set out hereinbefore)]:-

(a) <u>Tablet I</u>                             <u>mg/tablet</u>

Compound X.................................... 100

Lactose Ph.Eur............................. 182.75

Croscarmellose sodium....................... 12.0

Maize starch paste (5% w/v paste)........... 2.25

Magnesium stearate.......................... 3.0


(b) <u>Tablet II</u>                            <u>mg/tablet</u>

Compound X.................................... 50

Lactose Ph.Eur............................. 223.75

Croscarmellose sodium....................... 6.0

Maize starch............................... 15.0

Polyvinylpyrrolidone (5% w/v paste)......... 2.25

Magnesium stearate.......................... 3.0


(c) <u>Tablet III</u>                          <u>mg/tablet</u>

Compound X.................................... 1.0

Lactose Ph.Eur.............................. 93.25

Croscarmellose sodium....................... 4.0

Maize starch paste (5% w/v paste)........... 0.75

Magnesium stearate.......................... 1.0


(d) <u>Capsule</u>                              <u>mg/capsule</u>

Compound X................................... 10 mg

Lactose Ph.Eur*............................ 488.5

Magnesium stearate ......................... 1.5


(e) <u>Injection I</u>                        <u>(50 mg/ml)</u>

Compound X ................................. 5.0% w/v

1M Sodium hydroxide solution ............. 15.0% v/v

0.1M Hydrochloric acid

   (to adjust pH to 7.6)

Polyethylene glycol 400.................. 4.5% w/v

Water for injection to 100%

(f) <u>Injection II</u>                              (<u>10 mg/ml</u>)

Compound X ............................... 1.0% w/v

Sodium phosphate BP .................... 3.6% w/v

0.1M Sodium hydroxide solution .......... 15.0% v/v

Water for injection to 100%

(g) <u>Injection III</u>                    (<u>1mg/ml, buffered to pH6</u>)

Compound X ........................... 0.1% w/v

Sodium phosphate BP .................. 2.26% w/v

Citric acid .......................... 0.38% w/v

Polyethylene glycol 400 .............. 3.5% w/v

Water for injection to 100%

(h) <u>Aerosol I</u>                                    <u>mg/ml</u>

Compound X ........................... 10.0

Sorbitan trioleate ................... 13.5

Trichlorofluoromethane ............... 910.0

Dichlorodifluoromethane .............. 490.0

(i) <u>Aerosol II</u>                                   <u>mg/ml</u>

Compound X ............................... 2.5

Soya lecithin ............................ 2.7

Trichlorofluoromethane ................... 67.5

Dichlorodifluoromethane .................. 1086.0

Dichlorotetrafluoroethane ................ 191.6

<u>Note</u>

The above formulations may be obtained by conventional procedures well known in the pharmaceutical art. The tablets (a)-(c) may be enteric coated by conventional means, for example to provide a coating of cellulose acetate phthalate. The aerosol formulations (h)-(i) may be used in conjunction with standard, metered dose aerosol dispensers, and the suspending agents sorbitan trioleate and soya lecithin may be replaced by an alternative suspending agent such as sorbitan monooleate, sorbitan sesquioleate, polysorbate 80, polyglycerol oleate or oleic acid.

**Example 17**

The following illustrate representative pharmaceutical dosage forms containing both of the two active ingredients of the synergistic agent of the invention [hereinafter compounds [1] and I are used according to the abbreviations set out hereinbefore; alternatively compound [1] may be replaced by any one of compounds [2] to [5] and compound I may be replaced by compound F or compound D]:-

(a) Tablet I                                       mg/tablet

    Compound [1].................................. 100
    Compound I................................... 100
    Lactose Ph.Eur............................... 182.75
    Croscarmellose sodium........................ 12.0
    Maize starch paste (5% w/v paste)............ 2.25
    Magnesium stearate........................... 3.0


(b) Tablet II                                      mg/tablet

    Compound [1].................................. 50
    Compound I................................... 50
    Lactose Ph.Eur............................... 223.75
    Croscarmellose sodium........................ 6.0
    Maize starch................................. 15.0
    Polyvinylpyrrolidone (5% w/v paste).......... 2.25
    Magnesium stearate........................... 3.0


(c) Tablet III                                     mg/tablet

    Compound [1].................................. 1.0
    Compound I................................... 1.0
    Lactose Ph.Eur............................... 92.25
    Croscarmellose sodium........................ 4.0
    Maize starch paste (5% w/v paste)............ 0.75
    Magnesium stearate........................... 1.0


(d) Capsule                                        mg/capsule

    Compound [1].................................. 10 mg
    Comound I.................................... 10 mg
    Lactose Ph.Eur .............................. 478.5
    Magnesium stearate .......................... 1.5

(e)  Injection I ·                                              (50 mg/ml)

Compound [1]................................      2.5% w/v

Compound I.................................      2.5% w/v

1M Sodium hydroxide solution ...............     15.0% v/v

0.1M Hydrochloric acid

(to adjust pH to 7.6)

Polyethylene glycol 400....................      4.5% w/v

Water for injection to 100%


(f)  Injection II                                              (10 mg/ml)

Compound [1]................................      0.5% w/v

Compound I.................................      0.5% w/v

Sodium phosphate BP ........................      3.6% w/v

0.1M Sodium hydroxide solution .............     15.0% v/v

Water for injection to 100%


## Claims

1.  A synergistic agent for the treatment of inflammatory or arthritic conditions comprising a 5-lipoxygenase inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
in conjunction or admixture with a non-steroidal anti-inflammatory agent.

2.  A synergistic agent as claimed in claim 1 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

3.  A synergistic agent as claimed in claim 1 or claim 2 wherein the non-steroidal anti-inflammatory agent is selected from indomethacin, flurbiprofen and diclofenac.

4.  A synergistic agent as claimed in any of claims 1 to 3 wherein the molar ratio of the 5-lipoxygenase inhibitor and the non-steroidal anti-inflammatory agent is in the range 10:1 to 1:10.

5.  A pharmaceutical composition for the synergistic treatment of inflammatory or arthritic conditions which comprises a 5-lipoxygenase inhibitor inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
in conjunction or admixture with a non-steroidal anti-inflammatory agent, and a pharmaceutically-acceptable diluent or carrier.

6.  A pharmaceutical composition as claimed in claim 5 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

7. A pharmaceutical composition as claimed in claim 5 or claim 6 wherein the non-steroidal anti-inflammatory agent is selected from indomethacin, flubiprofen and diclofenac.

8. A pharmaceutical composition as claimed in any of claims 5 to 7 wherein the molar ratio of the 5-lipoxygenase inhibitor and the non-steroidal anti-inflammatory agent is in the range 10:1 to 1:10.

9. The use of a 5-lipoxygenase inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl)-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
in conjunction or admixture with a non-steroidal anti-inflammatory agent in the manufacture of an agent for the synergistic treatment of inflammatory or arthritic disease.

10. A use as claimed in claim 9 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

**Claims for the following Contracting States: ES, GR.**

1. A process for the preparation of a synergistic agent for the treatment of inflammatory or arthritic conditions characterised by bringing into conjunction or admixture a 5-lipoxygenase inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
and a non-steroidal anti-inflammatory agent.

2. A process as claimed in claim 1 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

3. A process as claimed in claim 1 wherein the non-steroidal anti-inflammatory agent is selected from indomethacin, flurbiprofen and diclofenac.

4. A process as claimed in claim 1 wherein the molar ratio of the 5-lipoxygenase inhibitor and the non-steroidal anti-inflammatory agent is in the range 10:1 to 1:10.

5. A process for the preparation of a pharmaceutical composition for the synergistic treatment of inflammatory or arthritic conditions characterised by bringing into conjunction or admixture a 5-lipoxygenase inhibitor inhibitor selected from
6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
a non-steroidal anti-inflammatory agent and a pharmaceutically-acceptable diluent or carrier.

6. A process as claimed in claim 5 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

7. A process as claimed in claim 5 wherein the non-steroidal anti-inflammatory agent is selected from indomethacin, flubiprofen and diclofenac.

8. A process as claimed in claim 5 wherein the molar ratio of the 5-lipoxygenase inhibitor and the non-steroidal anti-inflammatory agent is in the range 10:1 to 1:10.

9. The use of a 5-lipoxygenase inhibitor selected from

6-[(3-fluoro-5-[4-methoxy-3,4,5,6-tetrahydro-2H-pyran-4-yl]phenoxy)methyl]-1-methyl-2-quinolone,
4-[5-fluoro-3-(4-methyl-3-oxo-2,3-dihydro-4H-1,4-benzoxazin-7-ylthio)phenyl]-4-methoxytetrahydropyran,
4-methoxy-4-[5-(1-methyl-2-oxo-1,2,3,4-tetrahydroquinolin-6-ylthio)thien-2-yl]tetrahydropyran,
4-[5-fluoro-3-(1-methyl-2-oxo-1,2,3,4-tetrahydroquinolin-6-ylthio)phenyl]-4-methoxytetrahydropyran and
4-[5-fluoro-3-(4-(4-fluoro-alpha,alpha-difluorobenzyl)phenylthio)phenyl]-4-methoxytetrahydropyran,
in conjunction or admixture with a non-steroidal anti-inflammatory agent in the manufacture of an agent
for the synergistic treatment of inflammatory or arthritic disease.

10. A use as claimed in claim 9 wherein the 5-lipoxygenase inhibitor is 6-[(3-fluoro-5-[4-methoxy-3,4,5,6-tetrahydro-2H-pyran-4-yl]phenoxy)methyl]-1-methyl-2-quinolone.

11. A use as claimed in claim 9 or claim 10 wherein the non-steroidal anti-inflammatory agent is selected from indomethacin, flurbiprofen and diclofenac.

12. A use as claimed in any one of claims 9 to 11 wherein the molar ratio of the 5-lipoxygenase inhibitor and the non-steroidal anti-inflammatory agent is in the range 10:1 to 1:10.